# EUROPEAN PATENT SPECIFICATION

(11) **EP 0 863 862 B1**
(45) Date of publication and mention of the grant of the patent: **09.01.2002**
(21) Application number: 96926834.1
(22) Date of filing: 31.07.1996
(51) Int. Cl.: C07C 17/278, C07C 17/275, C07C 17/20

(54) **PROCESS FOR THE MANUFACTURE OF HALOCARBONS**
VERFAHREN ZUR HERSTELLUNG VON HALOGENKOHLENWASSERSTOFFEN
PROCEDE DE FABRICATION D'HYDROCARBURES HALOGENES

(30) Priority: 01.08.1995 US 1702 P
(43) Date of publication of application: 16.09.1998
(73) Proprietor: E.I. DU PONT DE NEMOURS AND COMPANY, Wilmington Delaware 19898 (US)
(72) Inventor: BAKER, Ralph, Thomas, Los Alamos, NM 87544 (US); PETROV, Viacheslav Alexandrovich, Hockessin, DE 19707-2401 (US); RAO, Velliyur, Nott, Mallikarjuna, Wilmington, DE 19809-1213 (US)
(74) Representative: Kuhnen & Wacker
(86) International application number: US9612548
(87) International publication number: WO9705090

(56) References cited:
- WO-A-95/04021
- WO-A-95/04022
- GB-A- 2 188 929
- US-A- 5 446 217

## Description

This invention relates to a process for the manufacture of halogenated alkanes by the catalytic reaction of haloalkanes with halogenated olefins.

### BACKGROUND

The catalyzed radical addition of haloalkanes to olefins is a well known reaction. Typically, however, when a haloalkane (e.g., AB, where A is a substituted carbon atom and B is a halogen other than fluorine) is added to an olefin (e.g., CH₂=CHR) to form the saturated adduct (e.g., CH₂ACHBR), the products (i.e., halogenated addition compounds) also include varying amounts of telomers (e.g., A(CH₂CHR)ₙB, where n is equal to 2 or more). For example, Canadian Pat.. No. 2,073,533 discloses a process for the manufacture of CCl₃CH₂CCl₃ by reacting carbon tetrachloride with vinylidene chloride using copper catalysts in acetonitrile. The selectivity for CCl₃CH₂CCl₃ with respect to converted vinylidene chloride was 87%. It has been shown in the art that the major by-product is the C₅ telomer, CCl₃(CH₂CCl₂)₂Cl.

WO-A 95/04021 and WO-A 95/04022 describe processes for the preparation of hydrofluorocarbons, wherein vinylidene chloride is reacted with CCl₄ in the presence of cupric or cuprous salts or a mixture thereof as catalyst and in the presence of amines like alkanol amines, alkyl amines or pyridine as co-catalysts.

The halogenated adducts are useful intermediates for the production of fluoroalkanes, particularly, hydrofluoroalkanes. These latter compounds are useful as refrigerants, fire extinguishants, heat transfer media, gaseous dielectrics, sterilant carriers, polymerization media, particulate removal fluids, carrier fluids, buffing abrasive agents, displacement drying agents, propellants, foaming agents and power cycle working fluids. There is an interest in developing more efficient processes for the manufacture of hydrofluoroalkanes.

### SUMMARY OF THE INVENTION

A liquid phase process is provided in accordance with this invention for producing halogenated alkane adducts of the formula CAR¹R²CBR³R⁴ wherein R¹, R², R³, and R⁴ are each independently selected from the group consisting of H, Br, Cl, F, C₁-C₆ alkyl, CN, CO₂CH₃, CH₂Cl, and aryl (e.g., phenyl), provided that only two of R¹, R², R³, and R⁴ can be selected from C₁-C₆ alkyl, CN, CO₂CH₃, CH₂Cl, and aryl; A is selected from the group consisting of CX₃, CH₃₋ₐXₐ, CₙH_{(2n+1)-b}X_{b} and CH_{c}X_{2-c}R, where R is CₙH_{(2n+1)-b}X_{b} (e.g., CF₃ and CCl₂CF₃), each X is independently selected from the group consisting of Br, Cl and I, a is an integer from 0 to 3, n is an integer from 1 to 6, b is an integer from 1 to 2n + 1, and c is an integer from 0 to 1; and B is selected from the group consisting of Br, Cl and I; provided that (1) when A is CX₃ then only one of X is I, (2) when A is CH₃₋ₐXₐ, then each X is B and a is an integer from 1 to 2 when B is Br, a is 2 when B is Cl, and a is an integer from 0 to 2 when B is I, and (3) when A is CₙH_{(2n+1)-b}X_{b}, then each X is independently selected from Cl and F, and B is I. The process comprises contacting a halogenated alkane of the formula AB (where A and B are as indicated above) with an olefin of the formula CR¹R²=CR³R⁴ (where R¹, R², R³ and R⁴ are as indicated above) in the presence of a catalyst system containing (i) at least one catalyst selected from the group consisting of monovalent and divalent copper, and (ii) at least one promoter selected from the group consisting of pyridazines pyrazines, and aliphatic heterocyclic compounds which contain at least one carbon-nitrogen double bond in the heterocyclic ring.

This invention further provides a process for producing hydrofluoroalkanes (e.g., CF₃CH₂CHF₂). This process comprises (a) producing a hydrochlorofluoroalkane (e.g., CCl₃CH₂CHCl₂) by reacting a halogenated alkane of the formula AB (e.g., CCl₄) and an olefin of the formula CR¹R²=CR³R⁴ (e.g., CH₂=CHCl) as indicated above (provided that B and X are Cl and at least one of said reactants contains hydrogen), and (b) reacting the hydrochlorofluoroalkane produced in (a) with HF.

### DETAILED DESCRIPTION

The present invention provides a liquid phase process for the manufacture of halogenated alkanes of the formula CAR¹R²CBR³R⁴ by contacting a corresponding halogenated alkane, AB, with a corresponding olefin, CR¹R²=CR³R⁴, in the presence of a copper catalyst (Cu⁺ and/or Cu⁺⁺) and a promoter (containing a C=N ring bond).

Examples of halogenated alkanes of the formula AB, where A and B are as defined above, include, CBrCl₃, CBrF₃, CCl₄, CCl₃F, CCl₂F₂, CF₃I, CCl₂FCCl₂F, CCl₃CF₃, CCl₃(CF₂CF₂)_{q}Cl (where q is an integer from 1 to 6), CCl₃CH₂CF₃, CCl₃CF₂CF₃, CCl₃CH₂CCl₃, CF₃CF₂I and CF₃CF₂CF₂I.

Examples of olefins of the formula CR¹R²=CR³R⁴, where R¹, R², R³ and R⁴ are as defined above, include, CF₂=CF₂, CF₂=CClF, CF₂=CCl₂, CClF=CClF, CClF=CCl₂, CF₂=CHF, CF₂=CH₂, CHF=CHF, CHF=CH₂, CH₂=CH₂, CH₂=CHCH₃, CH₂=CHCF₃, CH₂=CFCF₃, CH₂=CHCl, CH₂=CCl₂, CHCl=CHCl, CHCl=CCl₂, CH₂=CHCl, CH₂=CHCH₂Cl, CH₂=CHAryl (e.g., CH₂=CHC₆H₅), CH₂=CHCO₂CH₃, CH₂=C(CH₃)CO₂CH₃, CH₂=CHCO₂C₂H₅, and CH₂=C (CH₃) CO₂C₂H₅.

The addition of halogenated alkanes to alkenes (i.e., olefins) to form the corresponding adducts is catalyzed by copper compounds in the +1 or +2 oxidation state. Preferred copper compounds for the process of this invention include copper(I) chloride, copper(II) chloride, copper(I) bromide, copper(II) bromide, copper(I) iodide, copper(II)acetate and copper(II) sulfate. The catalysts are preferably anhydrous; and preferably, the addition is done under substantially anhydrous conditions in the substantial absence of oxygen. Without wishing to be bound by theory, it is believed that the effect of the catalyst is to enhance the yield of the 1:1 addition product (i.e., the adduct) of the halogenated alkanes to the alkene relative to higher molecular weight telomers that are known in the art.

Suitable promoters for use in the catalyst system include those selected from the group consisting of imidazoles, imidazolines, oxadiazoles, oxazoles, oxazolines, isoxazoles, thiazoles, thiazolines, pyrrolines, trihydropyrimidines, pyrazoles, triazoles, triazolium salts, isothiazoles, tetrazoles, tetrazolium salts, thiadiazoles, pyridazines, pyrazines, oxazines and dihydrooxazine. Preferred promoters include those selected from the group having Formula (I) or Formula (II) as follows: 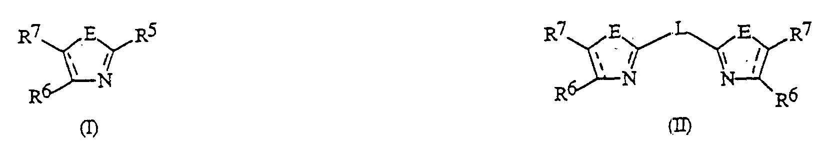 wherein E is selected from the group consisting of -O-, -S-, -Se-, -CH₂-, and -N(R⁸)-; R⁵ is selected from the group consisting of CH₃ and C₂H₅ (and is preferably CH₃); R⁶ and R⁷ are selected from the group consisting of H, CH₃, C₆H₅ (i.e., phenyl), CH₂C₆H₅, CH(CH₃)₂, and fused phenyl; L is selected from the group consisting of -O-, -S-, -Se-, -NR⁸-, -C₆H₄-,2,6-pyridyl, -OC₆H₄-C₆H₄O-, -CH₂CH₂OCH₂CH₂-, and -(CH₂)ₚ- where p is an integer from 0 to 6; and each R⁸ is selected from the group consisting of H and CₘH₂ₘ₊₁ where m is an integer from 1 to 6. The bond between each pair of carbon atoms respectively attached to R⁶ and R⁷ (as represented by the dashed bond lines in Formula (I) and Formula (II)) can be either a single or a double bond. Of note are compounds of Formula (II) which are optically active.

The reaction is done in the liquid phase, normally in the presence of solvents such as acetonitrile, dimethyl sulfoxide, dimethyl formamide, tetrahydrofuran, isopropanol, t-butanol, polyethers of the formula R⁹O(CH₂CH₂O)ᵣR⁹ where each R⁹ is independently selected from the group consisting of H, CH₃ and C₂H₅ and r is an integer from 1 to 4,-esters of formula R¹⁰CO₂R¹⁰ where each R¹⁰ is independently selected from C₁-C₆ alkyl groups and mixtures thereof; acetonitrile being preferred. Also of note are systems wherein the solvent divides the reaction mixture into two liquid phases. Reference is made to U.S. Patent Application No. 60/001,702 [CR-9789-P1] a priority document for PCT International Publication No. WO 97/05089 for further disclosure relating to such solvent systems.

The catalyst system comprising the copper compound and promoter as disclosed above can be prepared in the solvent in advance in a suitable mixing vessel, and then added to the reaction mixture. Alternatively, the individual components of the catalyst system can be added individually to the reactor. Of note are embodiments where the reaction is accomplished in a homogeneous system (i.e., where the catalyst is dissolved).

Telomer formation can be somewhat controlled by manipulating reaction variables such as the molar ratio of halogenated alkane, AB, to olefin, CR¹R²=CR³R⁴ or by adding the olefin to the halogenated alkane. Higher molar ratios of AB:CR¹R²=CR³R⁴ and dilution of the olefin reduce telomer formation. However, for the addition of CCl₄ to CH₂=CCl₂ the highest ratio of the C₃ adduct CCl₃CH₂CCl₃ to the C₅ adduct was reported to be 9:1 (see Belbachir et al., Makromol. Chem.1984, 185, 1583-1595).

The amount of catalyst used in the reaction of this invention is typically at least about 5 mmol, and preferably from about 10 mmol to 100 mmol, per mole of olefin, CR¹R²=CR³R⁴, used.

The amount of halogenated alkane starting material used in the reaction of this invention is typically at least about 1 mmol, and preferably from about 2 mmol to 10 mmol, per mmol of alkene used.

The amount of promoter used in the reaction of this invention is typically at least an amount sufficient to provide 2 mmol of heterocyclic ring which contains carbon-nitrogen double bonding per mmol of copper catalyst. For example, typically at least about 2 mmol of Formula (I) promoter or at least about 1 mmol of Formula (II) promoter, and preferably from about 4 mmol to 10 mmol of Formula (I) promoter or from about 2 mmol to 5 mmol of Formula (II) promoter is used per mmol of copper catalyst used.

The process of the present invention is suitably conducted at a temperature in the range of from about 50°C to 150°C, preferably from about 80°C to about 130°C.

The pressure of the process is not critical and can be subatmospheric, atmospheric or superatmospheric, preferably, superatmospheric. The reaction products may be separated by conventional techniques such as distillation.

Of note is the embodiment where AB is CCl₄ and CR¹R²=CR³R⁴ is CH₂=CCl₂. The isolated 1,1,1,3,3,3-hexachloropropane adduct can then be reacted with hydrogen fluoride to produce CF₃CH₂CF₃ (e.g., as disclosed in U.S. Pat. No. 5,414,165).

The reaction zone and its associated feed lines, effluent lines and associated units should be constructed of materials resistant to corrosion. Typical materials of construction include steel reactors lined with poly(tetrafluoroethylene) or glass and glass reactors.

The addition compounds that comprise the products of this invention are useful as intermediates for the formation of hydrofluoroalkanes. These addition compounds can be reacted with hydrogen fluoride in either the liquid or vapor phase in the presence of a suitable fluorination catalyst.

In the liquid phase, the addition compounds can be reacted with HF in the presence of catalysts selected from the halides of antimony, molybdenum, niobium, tantalum, tin and titanium, and mixtures thereof, preferably, antimony, niobium and tantalum. The temperature of the reaction can be in the range of 50°C to 175°C, preferably, 60°C to 150°C. The pressure is selected so that the the reaction medium is maintained in the liquid state, typically between 101 kPa and 5000 kPa, preferably, 1135 kPa to 3203 kPa. For example, 1,1,1,3,3,3-hexachloropropane (HCC-230fa) can be reacted with HF in the liquid phase using halides, fluorosulfonates or triflates of antimony, molybdenum, niobium, tantalum, tin or titanium, or mixtures thereof as catalysts to produce 1,1,1,3,3,3-hexafluoropropane (HFC-236fa). 1-Chloro-1,1,3,3,3-pentafluoropropane (HCFC-235fa) can also be prepared from HCC-230fa. HCFC-235fa can be hydrodechlorinated using a hydrodehalogenation catalyst to produce 1,1,1,3,3-pentafluoropropane (HFC-245fa). Palladium on acid-washed carbon is a preferred catalyst for the coversion of HCFC-235fa to HFC-245fa.

In another embodiment of this invention carbon tetrachloride can be reacted with vinyl chloride to produce the adduct 1,1,1,3,3-pentachloropropane (i.e., CCl₃CH₂CHCl₂ or HCC-240fa). CCl₃CH₂CHCl₂ can then be reacted with HF in the liquid phase using the process described above to produce CF₃CH₂CHF₂. The reaction products may be separated by conventional techniques such as distillation. Hydrofluorocarbons such as CF₃CH₂CHF₂ likely form azeotropes with HF; and conventional decantation/distillation may be employed if further purification of the hydrofluorocarbons is desired.

In the vapor phase, the addition compounds can be reacted with HF in the presence of catalysts comprising trivalent chomium. Catalysts prepared by pyrolysis of (NH₄)₂Cr₂O₇ to produce Cr₂O₃ and pretreated with HF and catalysts prepared by pretreating Cr₂O₃ having a surface area greater than about 200 m²/g with HF are preferred. The temperature of the reaction can be in the range of 200°C to 400°C, preferably, 250°C to 375°C. The pressure is not critical and is selected so that the reaction starting materials and products are maintained in the vapor state at the operating temperature. For example, it has recently been disclosed in U. S. Patent No. 5,414,165 that 1,1,1,3,3,3-hexafluoropropane may be prepared in high yield from 1,1,1,3,3,3-hexachloropropane by a vapor phase hydrofluorination process in the presence of a trivalent chromium catalyst.

Although the 1:1 addition compounds of the halogenated alkanes to the alkenes are the preferred products, the 2:1 adducts may also be useful intermediates.

Without further elaboration, it is believed that one skilled in the art can, using the description herein, utilize the present invention to its fullest extent. The following preferred specific embodiments are, therefore, to be construed as merely illustrative.

### EXAMPLE 1

### CCl₄ + CH₂=CCl₂ → CCl₃CH₂CCl₃

A suspension of 10 mg (0.1 mmol) CuCl in 4 mL of MeCN was treated with 2 equivalents of the promoter (i.e., ligand), except where noted otherwise, 114 mg of n-octane internal standard and 100 mg (1 mmol) of CH₂=CCl₂. To this mixture was added 1.54 g (10 mmol) CCl₄. The glass reaction vessel was then pressurized with 200 psi (1480 kPa) N₂ and heated at 100°C for 2 hr. The reaction mixture was analyzed by GC; results are summarized in Table 1, where %conversion is the molar% conversion of vinylidene chloride (i.e., CH₂=CCl₂), C₃:C₅ is the molar ratio of CCl₃CH₂CCl₃ to CCl₃CH₂CCl₂CH₂CCl₃, and the ligands used are shown in the Legend; monodentate ligands are numbered 1-12 and bidentates are labeled with letters A-F.

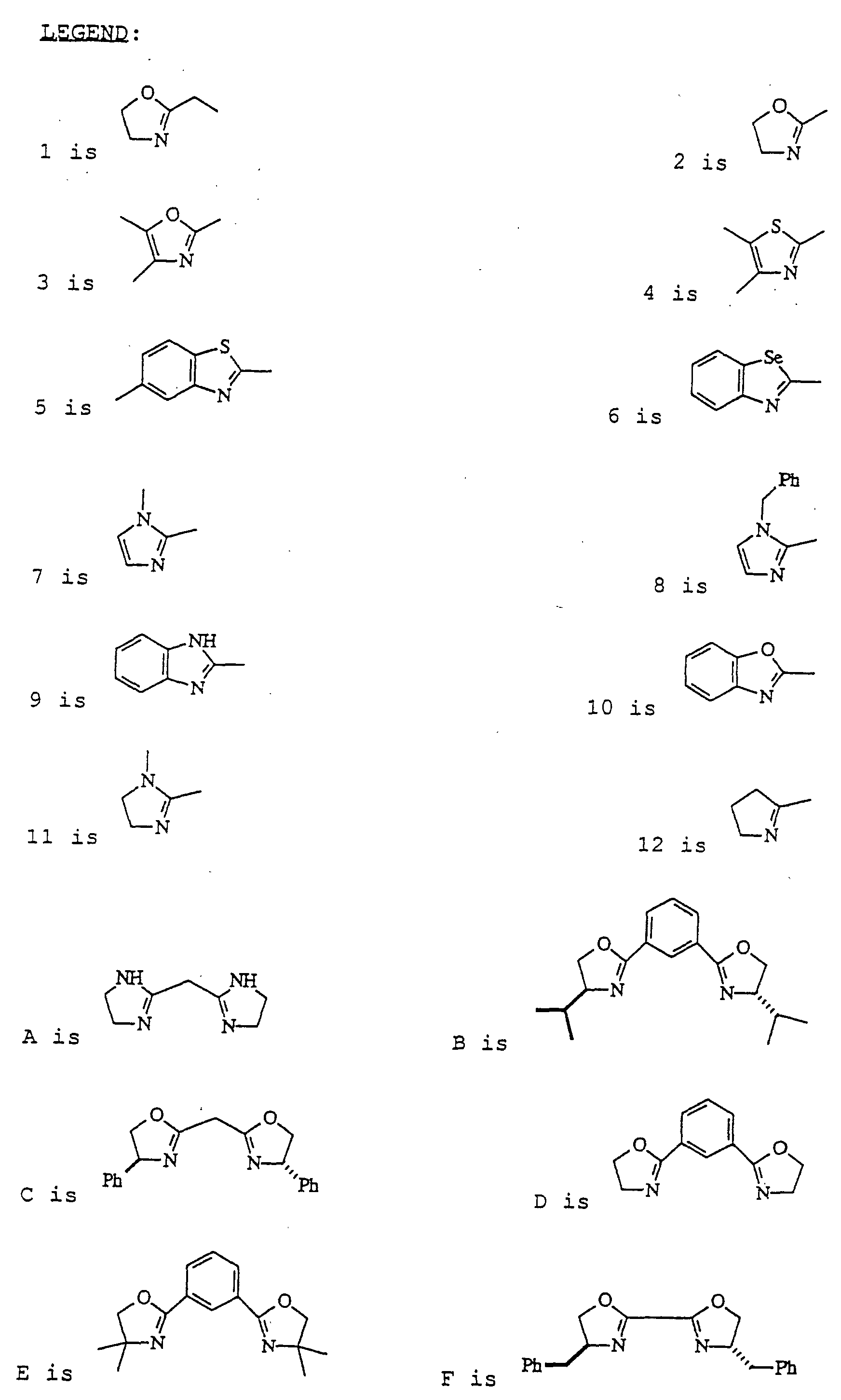

**Table 1**

| Run No. | Ligand | %Conv. | C₃:C₅ |
|---|---|---|---|
| 1 | A | 100 | 24 |
| 2 | B | 97 | 41 |
| 3 | C | 100 | 76 |
| 4 | 1^{a} | 100 | 19 |
| 5 | 1 | 100 | 32 |
| 6 | 1^{b} | 100 | 49 |
| 7 | 1^{c} | 100 | 66 |
| 8^{d} | 1 | 100 | 49 |
| 9 | 11 | 100 | >80 |
| 10 | 4 | 60 | >80 |
| 11 | 8 | 80 | >80 |
| 12 | 7 | 70 | 66 |
| 13 | 2 | 70 | 66 |
| 14 | 3 | <50 | 49 |
| 15 | 5 | >90 | 49 |
| 16 | 9 | 50 | 49 |
| 17 | 6 | 10 | 49 |
| 18 | D | >90 | 13 |
| 19 | E | 20 | 49 |
| 20 | 12 | >80 | 99 |

| | | | |
|---|---|---|---|
| a. One equivalent of the promoter was used. | | | |
| b. Four equivalents of the promoter was used. | | | |
| c. Ten equivalents of the promoter was used. | | | |
| d.The catalyst was CuCl₂. | | | |

### COMPARATIVE EXAMPLE

### No Promoter Added

The reaction was run in the same manner as that described in Example 1 with the following differences; the catalyst used was CuCl₂ (0.1 mmol) and no promoter was used.

After 2 hours at 100°C, no conversion of CH₂=CCl₂ was observed.

### EXAMPLE 2

### Reaction of CuCl and Ligand F

To a solution of 215 mg (0.67 mmol) bbbo (Legend, structure F) in 5 mL of MeCN was added 65 mg (0.66 mmol) of CuCl to give a strawberry red solution. The solution was filtered and 12 mL of Et₂O were added. After 20 hours, the resulting dark red crystals were filtered off, washed with Et₂O and dried to give 110 mg [Cu(bbbo)₂] [CuCl₂]. A second crop brought the total yield to 210 mg (75%). The structure was confirmed by X-ray crystallography.

### NMR monitoring of Cu-catalyzed addition of CCl₄ to alkenes

In one series of experiments, the effect of added ligand 2-Et-2-oxazoline was investigated using *trans*-β-Me-styrene as the alkene substrate. After 19 hr at 80°C, CuCl gave 14% conversion to PhCH(CCl₃)-CHClCH₃ with a diastereomeric ratio (DR) of 20. With 1 equiv of ligand/Cu, conversion was 91% and the DR was 10. With 2 and 4 equiv, conversions were 96 and 100% with DRs of 9 and 7, respectively. Under similar reaction conditions, use of [Cu(bbbo)₂][CuCl₂] as catalyst gave 92% conversion with a DR of 4.5. In another experiment with 4 equiv of 2-Et-2-oxazoline/Cu, conversion was 30% after only 30 min. at 80°C.

Using *trans-*PhCH₂CH=CHCH₃ as the alkene substrate, a comparison of 2-Et-2-oxazoline, ethanolamine, and diethylamine ligands showed that after 1.5 hr conversions were 18, 3 and 1 %, respectively.

### EXAMPLE 3

### Catalyst Selectivity and Lifetime Determination

These runs were done with only 0.5 mol% CuCl and a CCl₄/CH₂=CCl₂ ratio of one in order to differentiate promoters with respect to selectivity and lifetime. Reaction conditions: 4 mmol CCl₄, 4 mmol CH₂=CCl₂, 1 mL CD₃CN, 0.02 mmol CuCl and 0.08 mmol promoter (0.04 mmol for bidentate promoters). Reactions were heated in NMR tubes at 85°C. Results are shown in the following table.

| Run No. | Promoter | time (hr) | %conv | %C₃^{a} | %C₅^{b} | %C₇^{c} |
|---|---|---|---|---|---|---|
| 1 | 1 | 9 | 40 | 79 | 18 | 3 |
| | | 36 | 86 | 71 | 23 | 6 |
| | | 70 | 100 | 72 | 23 | 5 |
| | | | | | | |
| 2 | 2 | 9 | 47 | 84 | 16 | - |
| | | 36 | 72 | 92 | 8 | - |
| | | 70 | 100 | 88 | 12 | - |
| | | | | | | |
| 3 | 10 | 9 | 4 | 67 | 23 | - |
| | | 36 | 74 | 84 | 14 | 2 |
| | | 70 | 100 | 85 | 13 | 2 |
| | | | | | | |
| 4 | F | 9 | 22 | 94 | 6 | - |
| | | 22 | 60 | 92 | 8 | - |
| | | 70 | 100 | 94 | 6 | - |
| | | | | | | |
| 5 | C | 9 | 20 | 84 | 14 | 2 |
| | | 22 | 62 | 73 | 25 | 2 |
| | | 70 | 100 | 77 | 20 | 3 |
| | | | | | | |
| 6 | none | 72 | 97 | 80 | 18 | 2 |

| | | | | | | |
|---|---|---|---|---|---|---|
| a. C₃ is CCl₃CH₂CCl₃ | | | | | | |
| b. C₅ is CCl₃(CH₂CCl₂)₂Cl | | | | | | |
| c. C₇ is CCl₃(CH₂CCl₂)₃Cl | | | | | | |

Runs 1 and 5 show decreasing selectivity with time, indicative of limited promoter lifetime. Run 4 shows sustained high selectivity for 200 turnovers.

### EXAMPLE 4

### CCl₄ + CH₂=CCl₂ → CCl₃CH₂CCl₃

CuCl (0.1 g), 2-ethyl-2-oxazoline (0.4 g), deoxygenated CH₃CN (2 g), deoxygenated CCl₄ (16 g, 0.1 mol) and CH₂=CCl₂ (3 g, 0.031 mol) were charged into a 100 mL Pyrex® flask equipped with a Teflon® valve inside a dry box. The reaction mixture was kept at 80°C. After 4 hours it was analyzed by GC. The conversion of CH₂=CCl₂ was 100%, the yield of CCl₃CH₂CCl₃ was 92%, the selectivity of the reaction, defined as the ratio of CCl₃CH₂CCl₃ to CCl₃CH₂CCl₂CH₂CCl₃ was 97:3.

### EXAMPLE 5

### CCl₄ + CH₂=CF₂ → CCl₃CH₂CF₂Cl

A mixture of CCl₄ (52 g, 0.33 mol) CH₃CN (130 mL), CuCl (0.1 g) and 2-ethyl-2-oxazoline (0.4 g), prepared inside a dry box, was loaded into a 400 mL Hastelloy™ C nickel alloy shaker tube under N₂. The shaker tube was closed, cooled to -78°C, evacuated and CH₂=CF₂ (6.5 g, 0.1 mol) was added. The reaction mixture was kept at 130°C for 12 hours. The shaker tube was then unloaded; the reaction mixture was washed with water twice to remove CH₃CN, dried over P₂O₅ and the crude reaction mixture (40 g) analyzed by GC and ¹H and ¹⁹F NMR. The yield of CCl₃CH₂CF₂Cl was 70%, the selectivity of the reaction based on converted olefin was 81%.

### EXAMPLE 6

### CCl₄ + CHF=CF₂ → C₃HCl₄F₃

Example 5 was repeated using the same amounts of reagents except that CHF=CF₂ (8 g, 0.1 mol) was used instead of CH₂=CF₂. The isolated product (41 g) contained, based on ¹H, ¹⁹F NMR and GC, CCl₄ (51%), C₃Cl₄F₃H (29%, two isomers in a 7:3 ratio), C₅Cl₄F₆H₂ (8%, mixture of isomers) and CH₃CN (12%). The yield of isomeric propanes was 62%, the selectivity based on converted olefin was 60%.

### EXAMPLE 7

### CCl₄ + CHCl=CH₂ → CCl₃CH₂CHCl₂

Example 5 was repeated using the same amounts of reagents except that CHCl=CH₂ (7 g, 0.1 mol) was used instead of CH₂=CF₂. The isolated product (54 g) contained, based on ¹H, ¹⁹F NMR and GC, CH₃CN, CCl₄, CCl₃CH₂CHCl₂ and a small amount of CCl₃(CH₂CHCl)ₙCl (n = 2 and 3). The yield of CCl₃CH₂CHCl₂ was 69%, the selectivity based on converted olefin was 64%.

### EXAMPLE 8

### CCl₄ + CH₃CH=CH₂ → CCl₃CH₂CHClCH₃

Example 5 was repeated using the same amounts of reagents except that propylene (5 g, 0.1 mol) was used instead of CH₂=CF₂. The isolated product (54 g) contained, based on ¹H, ¹⁹F NMR and GC, CH₃CN, CCl₄, CCl₃CH₂CHClCH₃. The yield of CCl₃CH₂CHClCH₃ was 95%, the selectivity based on converted olefin was >95%.

### EXAMPLE 9

### CCl₄ + CF₂=CF₂ → CCl₃CF₂CF₂Cl

Example 5 was repeated using the same amounts of reagents except that tetrafluoroethylene (10 g, 0.1 mol) was used instead of CH₂=CF₂. The isolated product (45 g) contained, based on ¹⁹F NMR and GC, CCl₄ (66.5%), CCl₃CF₂CF₂Cl (23%), CCl₃(CF₂CF₂)₂Cl (6%), CCl₃(CF₂CF₂)₃Cl (2.6%), CCl₃(CF₂CF₂)₄Cl (1.4%), CCl₃(CF₂CF₂)₅Cl (0.6%). The yield of CCl₃CF₂CF₂Cl was 40%, the selectivity based on converted olefin was 65%.

### EXAMPLE 10

### CCl₃CF₃ + CH₂=CH₂ → CF₃CCl₂CH₂CH₂Cl

Example 5 was repeated using the same amounts of reagents except that ethylene (4 g, 0.1 mol) and CCl₃CF₃ (60 g, 0.3 mol) were used instead of CH₂=CF₂ and CCl₄. The isolated product (45 g) contained, based on ¹⁹F NMR and GC, CCl₃CF₃ (60%), CF₃CCl₂CH₂CH₂Cl (20%), CF₃CCl₂(CH₂CH₂)₂Cl (10%), CF₃CCl₂(CH₂CH₂)₃Cl (2%) and CH₃CN (8%). The yield of CF₃CCl₂CH₂CH₂Cl was 70%, the selectivity based on converted olefin was 63%.

## Claims

1. A liquid phase process for producing halogenated alkane adducts of the formula CAR¹R²CBR³R⁴ wherein
R¹, R², R³, and R⁴ are each independently selected from the group consisting of H, Br, Cl, F, C₁-C₆ alkyl, CN, CO₂CH₃, CH₂Cl, and aryl, provided that only two of R¹, R², R³, and R⁴ can be selected from C₁-C₆ alkyl, CN, CO₂CH₃, CH₂Cl, and aryl;
A is selected from the group consisting of CX₃, CH₃₋ₐXₐ, CₙH_{(2n+1)-b}X_{b} and CH_{c}X_{2-c}R, where R is CₙH_{(2n+1)-b}X_{b}, each X is independently selected from the group consisting of Br, Cl and I, a is an integer from 0 to 3, n is an integer from 1 to 6, b is an integer from 1 to 2n + 1, and c is an integer from 0 to 1; and
B is selected from the group consisting of Br, Cl and I;
provided that (1) when A is CX₃ then only one of X is I, (2) when A is CH₃₋ₐXₐ then each X is B and a is an integer from 1 to 2 when B is Br, a is 2 when B is Cl, and a is an integer from 0 to 2 when B is I, and (3) when A is CₙH_{(2n+1)-b}X_{b} then each X is independently selected from Cl and F and B is I, comprising:
contacting a halogenated alkane of the formula AB with an olefin of the formula CR¹R²=CR³R⁴ in the presence of a catalyst system containing (i) at least one catalyst selected from the group consisting of monovalent and divalent copper, and (ii) at least one promoter selected from the group consisting of pyridazines, pyrazines, and aliphatic heterocyclic compounds which contain at least one carbon-nitrogen double bond in the heterocyclic ring.

2. The process of Claim 1 wherein the halogenated alkane is selected from the group consisting of CBrCl₃, CBrF₃, CCl₄, CCl₃F, CCl₂F₂, CF₃I, CCl₂FCCl₂F, CCl₃CF₃, CCl₃CF₂CF₃, CCl₃CH₂CCl₃, CF₃CF₂I, CF₃CF₂CF₂I, CCl₃CH₂CF₃, and CCl₃(CF₂CF₂)_{q}Cl where q is an integer from 1 to 6.

3. The process of Claim 2 wherein the olefin is selected from the group consisting of CF₂=CF₂, CF₂=CClF, CF₂=CCl₂, CClF=CClF, CClF=CCl₂, CF₂=CHF, CF₂=CH₂, CHF=CHF, CHF=CH₂, CH₂=CH₂, CH₂=CHCH₃, CH₂=CHCF₃, CH₂=CFCF₃, CH₂=CHCl, CH₂=CCl₂, CHCl=CHCl, CHCl=CCl₂, CH₂=CHCl, CH₂=CHCH₂Cl, CH₂=CHAryl, CH₂=CHCO₂CH₃, CH₂=C(CH₃)CO₂CH₃, CH₂=CHCO₂C₂H₅, and CH₂=C(CH₃)CO₂C₂H₅.

4. The process according to any of claims 1 to 3 wherein the copper catalyst is selected from the group consisting of copper(I) chloride, copper(II) chloride, copper(I) bromide, copper(II) bromide, copper(I) iodide, copper(II) acetate and copper(II) sulfate.

5. The process according to any of claims 1 to 4 wherein the promoter is selected from the group consisting of imidazoles, imidazolines, oxadiazoles, oxazoles, oxazolines, isoxazoles, thiazoles, thiazolines, pyrrolines, trihydropyrimidines, pyrazoles, triazoles, triazolium salts, isothiazoles, tetrazoles, tetrazolium salts, thiadiazoles, pyridazines, pyrazines, oxazines and dihydrooxazine.

6. The process according to any of claims 1 to 4 wherein the promoter is selected from the group having the Formula (I) or Formula (II) 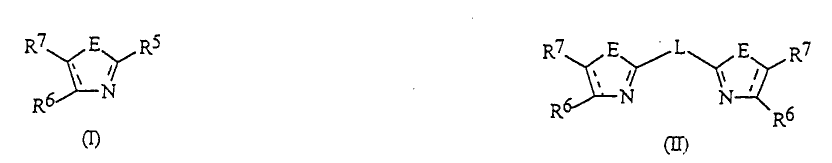 wherein E is selected from the group consisting of -O-, -S-, -Se-, -CH₂-, and -N(R⁸)-; R⁵ is selected form the group consisting of CH₃ and C₂H₅; R⁶ and R⁷ are selected from the group consisting of H, CH₃, C₆H₅, CH₂C₆H₅, CH(CH₃)₂, and fused phenyl; L is selected from the group consisting of -O-, -S-, -Se-, -N(R⁸)-, -C₆H₄-, 2,6-pyridyl, -OC₆H₄-C₆H₄O-, -CH₂CH₂OCH₂CH₂-, and -(CH₂)ₚ- where p is an integer from 0 to 6; and each R⁸ is selected from the group consisting of H and CₘH₂ₘ₊₁ where m is an integer from 1 to 6.

7. The process of Claim 6 wherein the promoter is a promoter of Formula II which is optically active.

8. The process according to any of claims 1 to 7 where the reaction is done in the presence of a solvent selected from the group consisting of acetonitrile, dimethyl sulfoxide, dimethyl formamide, tetrahydrofuran, isopropanol, t-butanol, polyethers of the formula R⁹O(CH₂CH₂O)ᵣR⁹ where each R⁹ is independently selected from the group consisting of H, CH₃ and C₂H₅ and r is an integer from 1 to 4, esters of the formula R¹⁰CO₂R¹⁰ where each R¹⁰ is independently selected from C₁-C₆ alkyl groups and mixtures thereof.

9. The process of Claim 8 wherein the solvent is acetonitrile.

10. The process according to any of the preceding claims wherein the reaction is accomplished in a homogenous system.

11. The process according to Claim 5 wherein the promoter is selected from the group consisting of imidazoles, imidazolines, oxadiazoles, oxazoles, oxazolines, isoxazoles, thiazoles, thiazolines, pyrrolines, trihydropyrimidines, pyrazoles, triazoles, triazolium salts, isothiazoles, tetrazoles, tetrazolium salts, thiadiazoles, oxazines and dihydrooxazine.

12. A process for producing a hydrofluoroalkane comprising:
(a) producing a hydrochlorofluoroalkane by reacting AB and CR¹R²=CR³R⁴ in accordance with the process of Claim 1, provided that B and X are Cl and at least one of AB and CR¹R²=CR³R⁴ contain hydrogen; and
(b) reacting the hydrochlorofluoroalkane produced in (a) with HF.

## Patentansprüche

1. Flüssigphasenverfahren zum Herstellen von halogenierten Alkanaddukten der Formel CAR¹R²CBR³R⁴, wobei
R¹, R², R³ und R⁴ jeweils unabhängig voneinander ausgewählt sind aus der Gruppe bestehend aus H, Br, Cl, F, C₁- bis C₆-Alkyl, CN, CO₂CH₃, CH₂Cl und Aryl, vorausgesetzt, daß nur zwei von R¹, R², R³ und R⁴ aus C₁- bis C₆-Alkyl, CN, CO₂CH₃, CH₂Cl und Aryl ausgewählt sein können;
A ausgewählt ist aus der Gruppe bestehend aus CX₃, CH₃₋ₐXₐ, CₙH_{(2n+1)-b}X_{b} und CH_{c}X_{2-c}R, wobei R für CₙH_{(2n+)-b}X_{b} steht, jedes X unabhängig ausgewählt ist aus der Gruppe bestehend aus Br, Cl und I, a eine ganze Zahl von 0 bis 3 ist, n eine ganze Zahl von 1 bis 6 ist, b eine ganze Zahl von 1 bis 2n+1 ist und c eine ganze Zahl von 0 bis 1 ist, und
B ausgewählt ist aus der Gruppe bestehend aus Br, Cl und I;
mit der Maßgabe: (1) wenn A CX₃ ist, dann ist nur ein X I, (2) wenn A CH₃₋ₐXₐ ist, dann ist jedes X B, und a ist eine ganze Zahl von 1 bis 2, wenn B Br ist, a ist 2, wenn B Cl ist, und a ist eine ganze Zahl von 0 bis 2, wenn B I ist, und (3) wenn A CₙH_{(2n+1)-b}X_{b} ist, dann ist jedes X unabhängig ausgewählt aus Cl und F und B ist I,
umfassend:
Inkontaktbringen eines halogenierten Alkans der Formel AB mit einem Olefin der Formel CR¹R²=CR³R⁴ in Anwesenheit eines Katalysatorsystems, das enthält: (i) mindestens einen Katalysator, ausgewählt aus der Gruppe bestehend aus einwertigem und zweiwertigem Kupfer und (ii) mindestens einen Promotor, ausgewählt aus der Gruppe bestehend aus Pyridazinen, Pyrazinen und aliphatischen heterocyclischen Verbindungen, die mindestens eine Kohlenstoff-Stickstoff-Doppelbindung im heterocyclischen Ring enthalten.

2. Verfahren nach Anspruch 1, wobei das halogenierte Alkan ausgewählt ist aus der Gruppe bestehend aus CBrCl₃, CBrF₃, CCl₄, CCl₃F, CCl₂F₂, CF₃I, CCl₂FCCl₂F, CCl₃CF₃, CCl₃CF₂CF₃, CCl₃CH₂CCl₃, CF₃CF₂I, CF₃CF₂CF₂I, CCl₃CH₂CF₃ und CCl₃(CF₂CF₂)_{q}Cl, wobei q eine ganze Zahl von 1 bis 6 ist.

3. Verfahren nach Anspruch 2, wobei das Olefin ausgewählt ist aus der Gruppe bestehend aus CF₂=CF₂, CF₂=CClF, CF₂=CCl₂. CClF=CClF, CClF=CCl₂, CF₂=CHF, CF₂=CH₂, CHF=CHF, CHF=CH₂, CH₂=CH₂, CH₂=CHCH₃, CH₂=CHCF₃, CH₂=CFCF₃, CH₂=CHCl, CH₂=CCl₂, CHCl=CHCl, CHCl=CCl₂, CH₂=CHCl, CH₂=CHCH₂Cl, CH₂=CHAryl, CH₂=CHCO₂CH₃, CH₂=C(CH₃)CO₂CH₃, CH₂=CHCO₂C₂H₅ und CH₂=C(CH₃)CO₂C₂H₅.

4. Verfahren nach einem der Ansprüche 1 bis 3, wobei der Kupferkatalysator ausgewählt ist aus der Gruppe bestehend aus Kupfer(I)-chlorid, Kupfer(II)-chlorid, Kupfer(I)-bromid, Kupfer(II)-bromid, Kupfer(I)-iodid, Kupfer(II)-acetat und Kupfer(II)-sulfat.

5. Verfahren nach einem der Ansprüche 1 bis 4, wobei der Promotor ausgewählt ist aus der Gruppe bestehend aus Imidazolen, Imidazolinen, Oxadiazolen, Oxazolen, Oxazolinen, Isoxazolen, Thiazolen, Thiazolinen, Pyrrolinen, Trihydropyrimidinen, Pyrazolen, Triazolen, Triazoliumsalzen, Isothiazolen, Tetrazolen, Tetrazoliumsalzen, Thiadiazolen, Pyridazinen, Pyrazinen, Oxazinen und Dihydrooxazinen.

6. Verfahren nach einem der Ansprüche 1 bis 4, wobei der Promotor ausgewählt ist aus der Gruppe mit der Formel (I) oder der Formel (II) 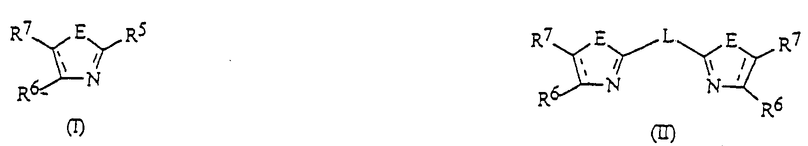 wobei E ausgewählt ist aus der Gruppe bestehend aus -O-, -S-, -Se-, -CH₂- und -N(R⁸)-, R⁵ ausgewählt ist aus der Gruppe bestehend aus CH₃ und C₂H₅; R⁶ und R⁷ ausgewählt sind aus der Gruppe bestehend aus H, CH₃, C₆H₅, CH₂C₆H₅, CH(CH₃)₂ und kondensiertem Phenyl; L ausgewählt ist aus der Gruppe bestehend aus -O-, -S-, -Se-, -N(R⁸)-, -C₆H₄-, 2,6-Pyridyl, -OC₆H₄-C₆H₄O-, -CH₂CH₂OCH₂CH₂- und -(CH₂)ₚ-, wobei p eine ganze Zahl von 0 bis 6 ist, und jedes R⁸ ausgewählt ist aus der Gruppe bestehend aus H und CₘH₂ₘ₊₁, wobei m eine ganze Zahl von 1 bis 6 ist.

7. Verfahren nach Anspruch 6, wobei der Promotor ein Promotor der Formel II ist, der optisch aktiv ist.

8. Verfahren nach einem der Ansprüche 1 bis 7, wobei die Reaktion in Anwesenheit eines Lösungsmittels durchgeführt wird, ausgewählt aus der Gruppe bestehend aus Acetonitril, Dimethylsulfoxid, Dimethylformamid, Tetrahydrofuran, Isopropanol, t-Butanol, Polyethem der Formel R⁹O(CH₂CH₂O)ᵣR⁹, wobei jedes R⁹ unabhängig ausgewählt ist aus der Gruppe bestehend aus H, CH₃ und C₂H₅ und r eine ganze Zahl von 1 bis 4 ist, Estern der Formel R¹⁰CO₂R¹⁰, wobei jedes R¹⁰ unabhängig ausgewählt ist aus C₁- bis C₆-Alkylgruppen und Mischungen davon.

9. Verfahren nach Anspruch 8, wobei das Lösungsmittel Acetonitril ist.

10. Verfahren nach einem der vorhergehenden Ansprüche, wobei die Reaktion in einem homogenen System durchgeführt wird.

11. Verfahren nach Anspruch 5, wobei der Promotor ausgewählt ist aus der Gruppe bestehend aus Imidazolen, Imidazolinen, Oxadiazolen, Oxazolen, Oxazolinen, Isoxazolen, Thiazolen, Thiazolinen, Pyrrolinen, Trihydropyrimidinen, Pyrazolen, Triazolen, Triazoliumsalzen, Isothiazolen, Tetrazolen, Tetrazoliumsalzen, Thiadiazolen, Oxazinen und Dihydrooxazin.

12. Verfahren zum Herstellen eines Hydrofluoralkans, umfassend:
(a) Herstellen eines Hydrochlorfluoralkans durch Umsetzen von AB und CR¹R²=CR³R⁴ gemäß dem Verfahren von Anspruch 1, mit der Maßgabe, daß B und X Cl sind und mindestens eine Verbindung von AB und CR¹R²=CR³R⁴ Wasserstoff enthält; und
(b) Umsetzen des in (a) erzeugten Hydrochlorfluoralkans mit HF.

## Revendications

1. Procédé en phase liquide pour la production d'adduits d'alcanes halogénés de formule CAR¹R²CBR³R⁴ dans laquelle
R¹, R², R³ et R⁴ sont chacun indépendamment choisis parmi le groupe consistant en H, Br, Cl, F, alkyle en C₁-C₆, CN, CO₂CH₃, CH₂Cl et aryle, à condition qu'uniquement deux des R¹, R², R³ et R⁴ puissent être choisis parmi alkyle en C₁-C₆, CN, CO₂CH₃, CH₂Cl et aryle ;
A est choisi parmi le groupe consistant en CX₃, CH₃₋ₐXₐ, CₙH_{(2n+1)-b}X_{b} et CH_{c}X_{2-c}R, où R est CₙH_{(2n+1)-b}X_{b}, chaque X est indépendamment choisi parmi le groupe consistant en Br, Cl et I, a est un entier de 0 à 3, n est un entier de 1 à 6, b est un entier de 1 à 2n+1, et c est un entier de 0 à 1 ; et
B est choisi parmi le groupe consistant en Br, Cl et I ;
à condition que (1) quand A est CX₃, uniquement un seul de X est I, (2) quand A est CH₃₋ₐXₐ, alors chaque X est B et a est un entier de 1 à 2 quand B est Br, a est 2 quand B est Cl, et a est un entier de 0 à 2 quand B est I, et (3) quand A est CₙH_{(2n+1)-b}X_{b}, alors chaque X est indépendamment choisi parmi Cl et F, et B est I, le procédé comprenant :
la mise en contact d'un alcane halogéné de formule AB avec une oléfine de formule CR¹R²=CR³R⁴ en présence d'un système de catalyseur contenant (i) au moins un catalyseur choisi parmi le groupe consistant en cuivre monovalent et divalent, et (ii) au moins un promoteur choisi parmi le groupe -consistant en pyridazines, pyrazines, et composés hétérocycliques aliphatiques qui contiennent au moins une double liaison carbone-azote dans l'hétérocycle.

2. Procédé selon la revendication 1 dans lequel l'alcane halogéné est choisi parmi le groupe consistant en CBrCl₃, CBrF₃, CCl₄, CCl₃F, CCl₂F₂, CF₃I, CCl₂FCCl₂F, CCl₃CF₃, CCl₃CF₂CF₃, CCl₃CH₂CCl₃, CF₃CF₂I, CF₃CF₂CF₂I, CCl₃CH₂CF₃, et CCl₃(CF₂CF₂)_{q}Cl (où q est un entier de 1 à 6).

3. Procédé selon la revendication 2 dans lequel l'oléfine est choisie parmi le groupe consistant en CF₂=CF₂, CF₂=CClF, CF₂=CCl₂, CClF=CClF, CClF=CCl₂, CF₂=CHF, CF₂=CH₂, CHF=CHF, CHF=CH₂, CH₂=CH₂, CH₂=CHCH₃, CH₂=CHCF₃, CH₂=CFCF₃, CH₂=CHCl, CH₂=CCl₂, CHCl=CHCl, CHCl=CCl₂, CH₂=CHCl, CH₂=CHCH₂Cl, CH₂=CHAryle, CH₂=CHCO₂CH₃, CH₂=C (CH₃) CO₂CH₃, CH₂=CHCO₂C₂H₅ et CH₂=C(CH₃) CO₂C₂H₅.

4. Procédé selon l'une quelconque des revendications 1 à 3 dans lequel le catalyseur de cuivre est choisi parmi le groupe consistant en chlorure de cuivre (I), chlorure de cuivre (II), bromure de cuivre (I), bromure de cuivre (II), iodure de cuivre (I), acétate de cuivre (II) et sulfate de cuivre (II).

5. Procédé selon l'une quelconque des revendications 1 à 4 dans lequel le promoteur est choisi parmi le groupe consistant en imidazoles, irnidazolines, oxadiazoles, oxazoles, oxazolines, isoxazoles, thiazoles, thiazolines, pyrrolines, trihydropyrimidines, pyrazoles, triazoles, sels de triazolium, isothiazoles, tétrazoles, sels de tétrazolium, thiadiazoles, pyridazines, pyrazines, oxazines et dihydrooxazines.

6. Procédé selon l'une quelconque des revendications 1 à 4 dans lequel le promoteur est choisi parmi le groupe ayant la formule (I) ou la formule (II) : 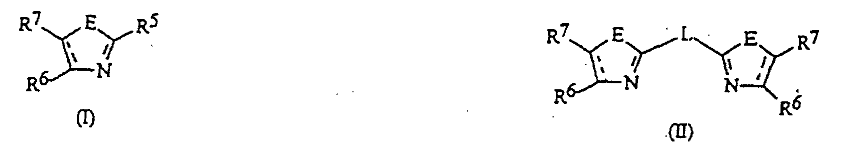 dans lesquelles E est choisi parmi le groupe consistant en -O-, -S-, -Se-, -CH₂- et N(R⁸)-; R⁵ est choisi parmi le groupe consistant en CH₃ et C₂H₅ ; R⁶ et R⁷ sont choisis parmi le groupe consistant en H, CH₃, C₆H₅, CH₂C₆H₅, CH(CH₃)₂ et phényle fusionné ; L est choisi parmi le groupe consistant en -O-, -S-, -Se-, -N(R⁸)-, -C₆H₄-, 2,6-pyridyle, -OC₆H₄-C₆H₄O-, -CH₂CH₂OCH₂CH₂- et -(CH₂)ₚ- où p est un entier de 0 à 6 ; et chaque R⁸ est choisi parmi le groupe consistant en H et CₘH₂ₘ₊₁ où m est un entier de 1 à 6.

7. Procédé selon la revendication 6 dans lequel le promoteur est un promoteur de formule II qui est optiquement actif.

8. Procédé selon l'une quelconque des revendications 1 à 7 où la réaction est faite en présence d'un solvant choisi parmi le groupe consistant en l'acétonitrile, le diméthylsulfoxyde, le diméthylformamide, le tétrahydrofuranne, l'isopropanol, le t-butanol, les polyéthers de formule R⁹O(CH₂CH₂O)ᵣR⁹ où chaque R⁹ est indépendamment choisi parmi le groupe consistant en H, CH₃ et C₂H₅ et r est un entier de 1 à 4, des esters de formule R¹⁰CO₂R¹⁰ où chaque R¹⁰ est indépendamment choisi parmi les groupements alkyle en C₁₋C₆ et les mélanges de ceux-ci.

9. Procédé selon la revendication 8 dans lequel le solvant est l'acétonitrile.

10. Procédé selon l'une quelconque des revendications précédentes dans lequel la réaction est accomplie dans un système homogène.

11. Procédé selon la revendication 5 dans lequel le promoteur est choisi parmi le groupe consistant en imidazoles, imidazolines, oxadiazoles, oxazoles, oxazolines, isoxazoles, thiazoles, thiazolines, pyrrolines, trihydropyrimidines, pyrazoles, triazoles, sels de triazolium, isothiazoles, tétrazoles, sels de tétrazolium, thiadiazoles, oxazines et dihydrooxazines.

12. Procédé pour la production d'un hydrofluoroalcane comprenant :
(a) la production d'un hydrochlorofluoroalcane par réaction de AB et de CR¹R²=CR³R⁴ selon le procédé de la revendication 1, à condition que B et X soient Cl et qu'au moins un de AB et de CR¹R²=CR³R⁴ contienne de l'hydrogène ; et
(b) la réaction de l'hydrochlorofluoroalcane produit en (a) avec HF.
